# EUROPÄISCHE PATENTSCHRIFT

(11) **EP 0 672 423 B1**
(45) Veröffentlichungstag und Bekanntmachung des Hinweises auf die Patenterteilung: **23.12.1998**
(21) Anmeldenummer: 95102467.8
(22) Anmeldetag: 22.02.1995
(51) Int. Cl.: A61L 2/06, A61L 2/24

(54) **Verfahren zur Sterilisation von Gebinden**
Method for sterilizing containers
Procédé de stérilisation de conteneurs

(30) Priorität: 16.03.1994 DE 4408839
(43) Veröffentlichungstag der Anmeldung: 20.09.1995
(73) Patentinhaber: GEA Till GmbH & Co., D-65830 Kriftel (DE)
(72) Erfinder: Till, Volker, Dipl. Ing., D-65719 Hofheim am Taunus (DE)
(74) Vertreter: KEIL & SCHAAFHAUSEN Patentanwälte

(56) Entgegenhaltungen:
- EP-A- 0 134 777
- WO-A-93/13880
- DE-A- 3 033 931

## Beschreibung

Die Erfindung betrifft ein Verfahren zur Sterilisation von Gebinden, insbesondere von Fässern wie Kegs, bei dem das Gebinde unter Druck, insbesondere Dampfdruck, gesetzt und dann verschlossen wird, und bei dem nach einer gewissen Haltezeit der im Gebinde herrschende Restdruck gemessen wird.

Bei den in der Getränkeindustrie verwendeten Gebinden, die üblicherweise als Fässer oder bei eingeschraubten Ventilen auch als Kegs bezeichnet werden, werden hohe Ansprüche an die Sauberkeit gestellt. Vor dem Befüllen werden daher die Gebinde üblicherweise mit heißem Dampf sterilisiert, der unter Druck in das Gebinde eingebracht wird und nach Verschließen des Gebindes für eine festgelegte Sterilisationszeit in dem Gebinde gehalten wird. Für die Qualität des Sterilisationsvorganges ist es dabei von großer Bedeutung, daß in dem Gebinde ein Überdruck herrscht, da sich bei Kondensation des Dampfes der Druck erniedrigt, so daß bei Entstehung eines Unterdrucks Umgebungsluft angesaugt und die Sterilität des Gebindeinneren beeinträchtigt wird. Zur Überprüfung des Sterilisationsvorgangs wird daher nach der Haltezeit gemessen, ob in dem Gebinde ein positiver Druck herrscht. Diese Haltezeit kann aber für jedes Gebinde sehr unterschiedlich sein, da es durch Störungen der Anlage oder andere äußere Faktoren zu Verzögerungen kommen kann, so daß einzelne Gebinde länger unter Druck gehalten werden als andere. Mit zunehmender Haltezeit nimmt aber der Druck im Gebinde aufgrund des Temperaturausgleichs mit der Umgebung ständig ab. Herkömmlicherweise wird bei der Überprüfung der Sterilisation lediglich festgestellt, ob ein Überdruck in dem Gebinde herrscht oder nicht. Bei Fehlen eines positiven Druckes wird das Gebinde nicht befüllt. Eine weitergehende Analyse des Sterilisationsvorgangs erfolgt nicht.

Aufgabe der Erfindung ist es daher, ein Verfahren der eingangs genannten Art derart weiterzubilden, daß eine genauere Aussage über die Qualität des Sterilisationsvorgangs ermöglicht wird.

Diese Aufgabe wird mit der Erfindung im wesentlichen dadurch gelöst, daß der Zeitpunkt des Schließens des Gebindes nach der Druckbeaufschlagung erfaßt und gespeichert wird, daß die Haltezeit bis zur Druckmessung ermittelt wird, und daß der gemessene Restdruckwert mit einem der ermittelten Haltezeit zugeordneten Druckwert in einer Standardabkühlkurve des untersuchten Gebindes verglichen wird. Da das Gebinde ständig Wärme an die Umgebung abgibt, nimmt der Druck im Gebinde stetig ab. Die Abfallkurve des Druckes läßt sich für ein ordnungsgemäßes Gebinde ermitteln und kann als Basis für die Analyse des einzelnen untersuchten Gebindes verwendet werden.

Es ist vorgesehen, daß die Druckbeaufschlagung mit heißem Dampf erfolgt. Die Dampftemperatur kann dabei unter Berücksichtigung der üblichen Haltezeit so gewählt werden, daß eine ausreichende Sterilisation gewährleistet ist.

Da die Druckabnahme in dem Gebindeinneren direkt von der Temperatur des Dampfes abhängt, wird bei einer bevorzugten Ausgestaltung der Erfindung die Differenz zwischen der Umgebungstemperatur und der Dampftemperatur bei der Druckbeaufschlagung ermittelt und eine bei einer etwa entsprechenden Temperaturdifferenz ermittelte Standardabkühlkurve zur Auswertung herangezogen.

Vorzugsweise wird für jeden Gebindetyp eine eigene Standardabkühlkurve ermittelt. Dadurch werden Einflüsse durch unterschiedliche Größen oder Wanddicken des Gebindes auf den Temperatur- und damit Druckabfall in dem Gebinde ausgeschlossen.

Eine schnelle und zuverlässige Analyse des Sterilisationsvorganges wird erfindungsgemäß dadurch erreicht, daß die Standardabkühlkurven in einer Datenverarbeitungseinrichtung gespeichert sind und daß die ermittelten Daten in der Datenverarbeitungseinrichtung ausgewertet werden.

Erfindungsgemäß wird das untersuchte Gebinde ausgesondert, wenn die gemessenen Daten außerhalb eines festgelegten Toleranzbereichs der zugeordneten Standardabkühlkurve liegen, da in diesem Fall von einer Undichtigkeit des Gebindes ausgegangen werden kann.

Weiterbildungen, Vorteile und Anwendungsmöglichkeiten der Erfindung ergeben sich aus der nachfolgenden Beschreibung eines Ausführungsbeispiels und der Zeichnung.

Es zeigen:
- Fig. 1: schematisch den Verfahrensablauf bei der erfindungsgemäßen Sterilisation von Gebinden, und
- Fig. 2: ein Druck-/Zeitdiagramm mit einer beispielhaften Standardabkühlkurve sowie der Abkühlkurve eines undichten Fasses.

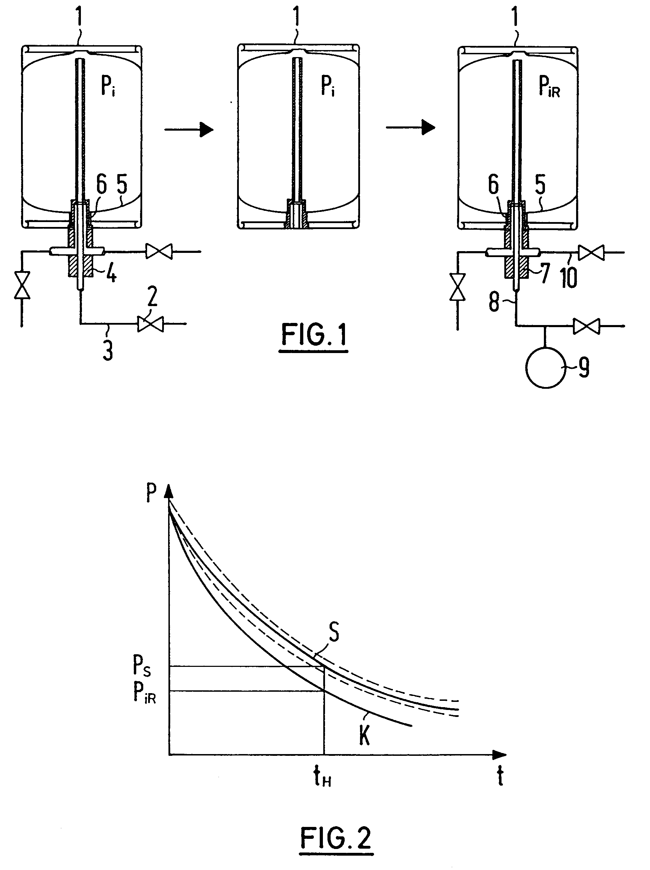

Bei der Sterilisation von Gebinden, insbesondere von Fässern wie Kegs, wird vor dem Befüllen eines Kegs 1 mit Getränkeflüssigkeit das Keg 1, wie im linken Teil von Fig. 1 dargestellt, mit Dampf beaufschlagt. Der Dampf wird über eine mit einem Ventil 2 absperrbare Zufuhrleitung 3 einem Behandlungskopf 4 zugeführt, auf welchen das Keg 1 mit einem in einer Bodenfläche 5 eingeschraubten Fitting 6 aufsetzbar ist.

Der Dampf wird bspw. mit einem Überdruck pᵢ von 2 bar in das Keg 1 eingebracht und weist eine dem Material des Kegs 1 und der üblichen Sterilisationsdauer entsprechende Temperatur auf. Das Keg besteht üblicherweise aus rostfreiem Stahl, so daß bei einer Sterilisationsdauer von etwa 30 Sek. der Dampf bspw. eine Temperatur von 127° C aufweisen muß.

Nach der Dampfbeaufschlagung wird, wie im mittleren Teil von Fig. 1 dargestellt, das Keg 1 verschlossen und für eine Haltezeit t_{H} bei dem in dem Keg 1 herrschenden Druck pᵢ gehalten. Aufgrund des Temperaturausgleichs mit der Umgebung sinkt der Druck pᵢ im Keg 1 stetig, bis er nach der Haltezeit t_{H} einen Restdruckwert p_{iR} erreicht.

Nach der Haltezeit t_{H} wird das Keg 1 wie in dem rechten Teil von Fig. 1 dargestellt, zum Befüllen mit Getränkeflüssigkeit auf einen Füllkopf 7 aufgesetzt. Der in dem Keg 1 herrschende Restdruck p_{iR} wird über eine Auslaßleitung 8 abgelassen, wobei der Restdruck p_{iR} mit einem Druckmeßgerät 9 gemessen wird. Nach positiver Auswertung kann das Keg 1 über eine Fülleitung 10 mit Produktflüssigkeit gefüllt werden.

Die Abkühlkurve des Kegs 1 während der Haltezeit t_{H} und damit die Abnahme des Drucks pᵢ in dem Keg 1 läßt sich experimentell ermitteln und in einer Datenverarbeitungseinrichtung speichern. Dabei werden den verschiedenen Haltezeiten t_{H} die jeweils gemessenen Druckwerte pᵢ zugeordnet. Beispielhaft ist eine solche Standardabkühlkurve S in Fig. 2 in einem Druck-/Zeitdiagramm dargestellt. Der Druck pᵢ nimmt dabei mit zunehmender Zeitdauer ab, wobei sich die Abkühlrate und damit das Gefälle der Abkühlkurve aufgrund des sinkenden Temperatur unterschieds zur Umgebung verringert.

Bei der Überprüfung des Sterilisationsvorgangs wird der Zeitpunkt der Beaufschlagung des Kegs 1 mit dem Dampfdruck pᵢ erfaßt und dem Keg 1 zugeordnet. Beim Öffnen des Kegs 1 vor dem Befüllen wird der Restdampfdruck p_{iR} in dem Keg 1 mit dem Druckmeßgerät 9 gemessen. Der gemessene Dampfdruckwert p_{iR} wird mit einem der ermittelten Haltezeit t_{H} seit Schließen des Kegs 1 zugeordneten Dampfdruckwert pₛ in der Standardabkühlkurve des Kegs 1 verglichen. Liegt der gemessene Dampfdruckwert p_{iR} außerhalb eines in Fig. 2 mit gestrichelten Linien dargestellten festgelegten Toleranzbereichs um die Standardabkühlkurve S, so kann davon ausgegangen werden, daß das untersuchte Keg 1 ein Loch oder eine beschädigte Dichtung aufweist, so daß zusätzlich zu dem Druckverlust durch Temperaturabstrahlung ein Masseverlust an die Umgebung erfolgte. Somit läßt sich durch das erfindungsgemäße Verfahren nicht nur der Sterilisationsprozeß sondern auch die Dichtigkeit des Kegs 1 überprüfen. In Fig. 2 ist unter Andeutung der der Haltezeit t_{H} zugeordneten Druckwerte P_{iR} und Pₛ beispielhaft eine Abkühlkurve K eines undichten Kegs dargestellt, die unterhalb der Standardabkühlkurve S verläuft.

Da die Druckabnahme in dem Keg 1 direkt von der Temperaturdifferenz zwischen dem Dampf und der Umgebung abhängt, wird die Dampftemperatur bei der Druckbeaufschlagung des Kegs 1 erfaßt und dem Keg 1 eine bei einer entsprechenden Temperatur ermittelte Standardabkühlkurve zugeordnet.

Da die Temperatur- und damit Druckabnahme auch von dem jeweiligen Gebindetyp abhängt, werden für jeden Gebindetyp eigene Standardabkühlkurven ermittelt.

Die gemessenen Druck-, Zeit- und Temperaturwerte werden an eine Datenverarbeitungseinrichtung übermittelt und dort mit den entsprechend ausgewählten Standardabkühlkurven verglichen. Somit ist eine automatische Verarbeitung der gewonnenen Daten möglich, die durch Kopplung mit der Steuerung der Abfüllanlage das Aussondern als undicht erkannter Kegs ermöglicht.

### Bezugszeichenliste:

- 1: Keg
- 2: Ventil
- 3: Zufuhrleitung
- 4: Behandlungskopf
- 5: Bodenfläche
- 6: Fitting
- 7: Füllkopf
- 8: Auslaßleitung
- 9: Druckmeßgerät
- 10: Fülleitung
- Pᵢ: Keginnendruck
- P_{iR}: Restdruck
- Pₛ: Druck gemäß Standardabkühlkurve
- t_{H}: Haltezeit
- K: Abkühlkurve eines undichten Kegs
- S: Standardabkühlkurve

## Patentansprüche

1. Verfahren zur Sterilisation von Gebinden, insbesondere von Fässern wie Kegs, bei dem das das Innere des Gebindes mit Sterilisationsmedium unter Druck (pᵢ), insbesondere Dampfdruck, gesetzt und dann verschlossen wird, und bei dem nach einer gewissen Haltezeit (t_{H}) der im Gebinde herrschende Restdruck (P_{iR}) gemessen wird, **dadurch gekennzeichnet,** daß der Zeitpunkt des Schließens des Gebindes nach der Druckbeaufschlagung erfaßt und gespeichert wird, daß die Haltezeit (t_{H}) bis zur Druckmessung ermittelt wird, und daß der gemessene Restdruckwert (P_{iR}) mit einem der ermittelten Zeitdauer zugeordneten Druckwert (p_{S}) in einer Standardabkühlkurve (S) des untersuchten Gebindes, die bei einer etwa der Differenz zwischen der Temperatur des beaufschlagenden Sterilisationsmediums und der Umgebungstemperatur entsprechenden Temperaturdifferenz ermittelt wurde, verglichen wird.

2. Verfahren nach Anspruch 1, **dadurch gekennzeichnet,** daß die Differenz zwischen der Umgebungstemperatur und der Dampftemperatur bei der Druckbeaufschlagung ermittelt wird, und daß eine bei einer etwa entsprechenden Temperaturdifferenz ermittelte Standardabkühlkurve (S) zur Auswertung herangezogen wird.

3. Verfahren nach einem der Ansprüche 1 oder 2, **dadurch gekennzeichnet,** daß für jeden Gebindetyp eigene Standardabkühlkurven (S) ermittelt werden.

4. Verfahren nach einem der Ansprüche 1 bis 3, **dadurch gekennzeichnet,** daß die Standardabkühlkurven (S) in einer Datenverarbeitungseinrichtung gespeichert sind, und daß die ermittelten Daten in der Datenverarbeitungseinrichtung ausgewertet werden.

5. Verfahren nach einem der Ansprüche 1 bis 4, **dadurch gekennzeichnet,** daß das untersuchte Gebinde ausgesondert wird, wenn die gemessenen Daten außerhalb eines festgelegten Toleranzbereichs der zugeordneten Standardabkühlkurve (S) liegen.

## Claims

1. Method of sterilising containers, in particular barrels such as kegs, in which the interior of the container is subjected to sterilisation medium under pressure (pᵢ), in particular steam pressure, and is then sealed, and in which the residual pressure (P_{iR}) prevailing in the container after a certain holding time (t_{H}) is measured, characterised in that the time at which the container is sealed after pressurisation is determined and stored, that the holding time (t_{H}) to the measurement of the pressure is determined and that the residual pressure value (P_{iR}) measured is compared with a pressure value (pₛ) associated with the time determined in a standard cooling curve (S) for the container being tested, determined at a temperature difference corresponding approximately to the difference between the temperature of the pressurising sterilisation medium and the ambient temperature.

2. Method according to claim 1, characterised in that the difference between the ambient temperature and the steam temperature during pressurisation is determined and that a standard cooling curve (S) determined at an approximately corresponding temperature difference is used for the evaluation.

3. Method according to one of claims 1 or 2, characterised in that separate standard cooling curves (S) are determined for each type of container.

4. Method according to one of claims 1 to 3, characterised in that the standard cooling curves (S) are stored in a data processor and that the data determined is evaluated in the data processor.

5. Method according to one of claims 1 to 4, characterised in that the container being tested is rejected if the data measured is outside a predetermined tolerance range for the associated standard cooling curve (S).

## Revendications

1. Procédé de stérilisation de récipients en particulier de fûts, tels que des tonnelets, selon lequel on applique une pression (Pᵢ) notamment une pression de vapeur, à l'intérieur du récipient chargé en agent de stérilisation, on ferme ledit récipient et on mesure la pression résiduelle (P_{iR}) dans le récipient après un temps de maintien (t_{H}) déterminé, caractérisé par le fait que l'on détecte l'instant de fermeture du récipient après application de la pression et on le mémorise, que l'on détermine le temps de maintien (t_{H}) jusqu'à la mesure de la pression et que l'on compare la valeur de pression résiduelle (P_{iR}) mesurée à une valeur de pression (P_{S}) correspondant au temps mesuré, lue sur une courbe de refroidissement type (S) pour le récipient examiné, établie pour une différence de température qui correspond sensiblement à la différence entre la température de l'agent de stérilisation utilisé et la température ambiante.

2. Procédé selon la revendication 1, caractérisé par le fait que l'on détermine la différence entre la température ambiante et la température de vapeur lors de l'application de la pression et que l'on utilise pour l'exploitation une courbe de refroidissement type (S) établie pour une différence de température sensiblement identique.

3. Procédé selon une des revendications 1 ou 2, caractérisé par le fait que l'on détermine des courbes de refroidissement type (S) propres pour chaque type de récipient.

4. Procédé selon une des revendications 1 à 3, caractérisé par le fait que l'on mémorise les courbes de refroidissement type (S) dans un système de traitement de données et par le fait que l'on exploite les données déterminées dans le système de traitement de données.

5. Procédé selon une des revendications 1 à 4, caractérisé par le fait que l'on isole le récipient examiné lorsque les données mesurées sont situées à l'extérieur d'une plage de tolérances déterminée de la courbe de refroidissement type (S).
